**Europäisches Patentamt**

**European Patent Office**

**Office européen des brevets**

(19)

(11) Veröffentlichungsnummer: **0 144 713 B1**

(12) ## EUROPÄISCHE PATENTSCHRIFT

(45) Veröffentlichungstag der Patentschrift: **24.07.91**

(51) Int. Cl.⁵: **G01N 21/17, A61B 5/00, G01N 13/00**

(21) Anmeldenummer: **84113091.7**

(22) Anmeldetag: **30.10.84**

---

(54) **Anordnung zur optischen Messung von Stoffkonzentrationen.**

---

(30) Priorität: **06.12.83 DE 3344019**

(43) Veröffentlichungstag der Anmeldung:
**19.06.85 Patentblatt 85/25**

(45) Bekanntmachung des Hinweises auf die
Patenterteilung:
**24.07.91 Patentblatt 91/30**

(84) Benannte Vertragsstaaten:
**AT BE CH FR GB IT LI LU NL SE**

(56) Entgegenhaltungen:
**EP-A- 0 083 703**
**EP-A- 0 091 046**
**EP-A- 0 095 074**
**DE-B- 2 508 637**
**DE-B- 2 632 710**

(73) Patentinhaber: **Max-Planck-Gesellschaft zur
Förderung der Wissenschaften e.V.
Bunsenstrasse 10
W-3400 Göttingen(DE)**

(72) Erfinder: **Opitz, Norbert, Dr. Dipl.-Phys.
Villigsterstrasse 6
W-5840 Schwerte 5(DE)**
Erfinder: **Lübbers, Dietrich W., Prof. Dr.
Rheinlanddamm 201 a
W-4600 Dortmund 1(DE)**
Erfinder: **Schrader, Bernhard, Prof. Dr.
Soniusweg 20
W-4300 Essen(DE)**

(74) Vertreter: **Krause, Walter, Dr. Dipl.-Ing.
Postfach 200 Singerstrasse 8
A-1010 Wien(AT)**

**Beschreibung**

Die Erfindung betrifft eine Anordnung zur optischen Messung von Stoffkonzentrationen.

In biologischen Messobjekten muessen haeufig Konzentrationen von Teilchenfraktionen nachgewiesen werden und dieser Nachweis wird ueberwiegend in waessrigen Loesungen vorgenommen.

Dabei sind die Stoereinfluesse der jeweils nicht interessierenden Teilchen oder des Wassers auf die zu messenden Teilchen meist sehr gross. Bei optischen Messungen wird haeufig auch die Messtrahlung gestoert, sodass quantitative Messungen in Frage gestellt sind.

Waehrend die durch Lumineszenz-oder Absorptionsindikatoren optisch messbaren Teilchenarten beispielsweise gemass DE-PS 2508637 durch Optoden -das sind optische Indikatoren enthaltende Indikatorraeume- messbar sind, ist die optische Messung von IR-aktiven Teilchen oder von Teilchen, fuer die keine Indikatoren vorhanden sind, wegen der oben angefuehrten Umstaende problematisch.

Auch ist die Verwendung von Lichtleitern aus der DE-OS 2632710 bei der Messung von Stoffkonzentrationen bekannt.

Die Erfindung hat sich deshalb die Aufgabe gestellt, die optische Messung von Teilchenkonzentrationen, die nicht durch Optoden messbar sind, zu verbessern.

Sie loest diese Aufgabe dadurch, dass

eine Anordnung zur optischen Messung von Stoffkonzentrationen mit

einer IR-Strahlungsquelle zur Erzeugung der Messstrahlung (Pp)

einem Messraum (M) aus einem, fuer die zu messenden Teilchen (Tim) selektiv permeirbaren und fuer die Messstrahlung (Pp) transparenten Stoff, der so angeordnet ist, dass eine Diffusion der zu messenden Teilchen (Tim) in den Messraum erfolgen kann,

einem Lichtleiter (L), der an seinen Seitenflaechen mit dem Messraum (M) umkleidet ist und der so angeordnet ist, dass die Messstrahlung (P) in den Lichtleiter (L) faellt und in ihm innerhalb des Grenzwinkels der Totalreflexion verlaeuft und

einem Fotoempfanger (F) zur Untersuchung der aus dem Lichtleiter (L) wieder austretenden Messstrahlung (Pp) '

vorgesehen ist.

Der Vorteil dieser Anordnung besteht darin, dass einmal durch die Selektivitat des Messraumes fuer die zu messenden diffundierenden Teilchen eine Separation von anderen, nicht zu messenden, aber stoerenden Teilchen ermoeglicht ist.

Ein weiterer Vorteil besteht darin, dass die Absorptionskurve fuer die Strahlung in den Faellen verschoben werden kann, in denen die den Messraum bildenden Molekuele auf die zu messenden Teilchen zurueckwirken, was dann die Verschiebung der Absorption bewirkt. Dadurch ergibt sich eine zusaetzliche Moeglichkeit der Abstimmung der Wellenlaengen von Strahler, Absorptionsbereich und Empfaenger aufeinander, oder aber der Arbeitsbereich kann aus einer im Messbereich liegenden Stoerbande verschoben werden.

Darueberhinaus kann bei Verwendung von Substanzen fuer den Messraum, die eine Bindung oder einen hohen Loeslichkeitskoeffizienten fuer die zu messenden Teilchen aufweisen, deren Konzentration gegenueber dem Messraum erhoeht und dadurch das Signal-Rauschverhaeltnis fuer die Messstrahlung verbessert werden.

Die als Messraum verwendbaren Stoffe muessen fuer die Messstrahlung transparent sein. Da eine solche Transparenz -beispielsweise mit Siliconen, PVC, Polystyrol, Polypropylen oder dergleichen- ohne weiteres auch fuer die IR-Messstrahlung moeglich ist, koennen nunmehr auch diffusionsfaehige Stoffe ohne spezifische Indikatoren aber mit ausgepraegter IR-Aktivitaet oder mit Ramanaktivitaet optisch vermessen werden. Das Narkosegas Halothan ist dafuer ein Beispiel.

Anordnungen nach der Erfindung eignen sich jedoch nicht nur fuer einfache Durchstrahlungen, sondern koennen auch als Lichtleiter oder Teile von Lichtleitern verwendet werden.

Damit eine Lichtleitung im Lichtleiter erfolgen kann, muss das transportierte Licht an den Seitenflächen des Lichtleiters innerhalb des Grenzwinkels der Totalreflexion verlaufen. Das Licht wird dann von der Grenzfläche reflektiert, aus physikalischen Gründen jedoch nicht ideal reflektiert, sondern es dringt eine kurze Strecke -etwa von der Grössenordnung einer Wellenlänge- in den Aussenraum ein. Wird dort ein Messraum nach der Erfindung angeordnet, dann durchsetzt die Messstrahlung diesen Messraum. Die effektive Dicke des Messraumes wird dabei durch die Messtrahlung und ihren Einstrahlungswinkel, also physikalisch bestimmt.

Da die Messstrahlung aus physikalischen Gründen nicht tiefer in den Messraum eindringen kann, sind auch Mittel zur optischen Entkopplung der Messstrahlung vom Messobjekt nicht mehr erforderlich. Dazu weist der Messraum die Dicke mehrerer Wellenlängen auf.

Ist die Löslichkeit des Messraumes oder die Bindung für die zu messende Teilchenart durch entsprechende Wahl des Stoffes, aus dem er besteht, gegenüber dem Messgut erhöht, dann stellt sich im Messraum eine erhöhte Konzentration für diese Teilchen ein und das Signal/Rauschverhältnis der Messungen ist dadurch verbessert.

Sollen mehrere Paramter oder Teilchenarten gemessen werden, ist eine Felderung des Messraumes vorgesehen, wobei die Indikatorfelder teilchenselektiv ausgebildet sind.

Ist die Membran hydrophob ausgebildet, dann werden beispielsweise die zu messenden Teilchen, die in den Indikatorraum diffundieren, vom Wasser getrennt. Hierdurch ist es beispielsweise möglich, $CO_2$-Konzentrationen oder Konzentrationen von Narkosegas (Halothan) im Blut oder anderen wässrigen Lösungen mittels IR-Strahlung zu messen. Das ist sonst nicht möglich, weil das Wasser eine sehr hohe IR-Absorption durch die sogenannten "Wasserbanden" hat, die IR-Messungen in Durchlicht oder Reflexion nicht gestatten.

Als Material für solche, dem Messraum zugeordneten Lichtleiter kann zum Beispiel Thalliumbromid-Jodid (KRS5), ZnSe, ZnS, Si, Ge oder, wo dies technisch möglich ist, kristallines heteropolares Salz wie CaF oder KBr oder NaCl verwendet werden.

Als Sperre für Wasserdampf können weitere Schichten, wie beispielsweise Tetrafluorethylen oder Mylar, über den Messraum gezogen sein. Oder es sind Schichten aus wasserunlöslichen Lösungsmitteln, wie beispielsweise Dioctylphtalat, vorgesehen.

Solche Mittel können entweder selbsthaftend sein oder mittels poröser Netzwerke, beispielsweise Teflonvliess (Milliporfilter) oder dergleichen immobilisiert sein.

Auch die Verwendung von aktivierten Polymerfolien, die beispielsweise carrierdotiert sein können, ist möglich. Dadurch wird eine bessere Selektivität für die zu messenden Teilchen erreicht.

In der Zeichnung, anhand derer die Erfindung weiter erläutert wird, zeigen:

Fig.1: Eine erste Messanordnung nach der Erfindung

Fig.2: Eine zweite Messanordnung nach der Erfindung

Fig.3: Eine dritte Messanordnung nach der Erfindung

Fig.4: Eine Teilanordnung nach der Erfindung im Schnitt

Fig.5: Einen Lichtleiter im Schnitt

Fig.6: Eine Mehrschichtanordnug nach der Erfindung

Fig.7: Einen als Tauchstab verwendbaren Lichtleiter

Fig.8: Eine Ausbildung mit gestrecktem Lichtleiter

Fig.9: Eine gefelderte Anordnung

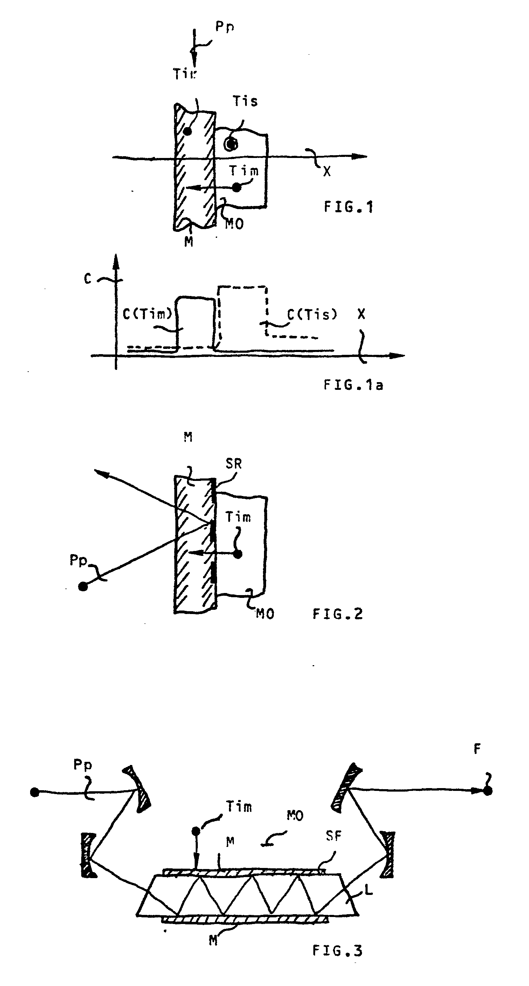

Nach Fig.1 verläuft die Messtrahlung Pp durch einen Messraum M, der mit einem Messobjekt MO in Wirkverbindung steht. Die Teilchenfraktion Tim diffundiert in den Messraum M ein und liegt, wenn die Löslichkeit a für die Teilchen Tim im Messraum M höher ist als im Messobjekt MO, in erhöhter Konzentration c(Tim) gemäss Fig.1a im Messraum M vor. Andererseits werden Teilchen Tis, die nicht in den Messraum M diffundieren können, -wie beispielsweise Wasser bei Verwendung lipophiler Messräume- vom Messraum M und damit von der Messung ausgeschlossen.

Die Konzentrationserhöhung im Messraum M kann, je nach Bindung oder Löslichkeit, bis zu drei Grössenordnungen betragen.

Wenn die Voraussetzung zur Messung im Durchlicht -transparentes Messobjekt MO-, nicht vorliegen, kann auch in Reflexion nach Fig. 2 gemessen werden. Dazu kann z.B. der Messraum M nach dem Messobjekt MO hin mit einer Teilreflexionsschicht SR abgeschlossen sein.

Zur Messung in sehr dünnen Schichten bildet gemäß der Erfindung nach Fig.3 der Messraum M eine Seitenfläche SF eines Lichtleiters L. Da die Messtrahlung Pp in einem Lichtleiter L unterhalb des Grenzwinkels der Totalreflexion verläuft, werden bei der Reflexion kurze Wege innerhalb des Messraumes M zurückgelegt, auf denen eine Einwirkung der Teilchen auf diese Strahlung erfolgen kann. Ein Photoempfänger F nimmt diese Strahlung auf.

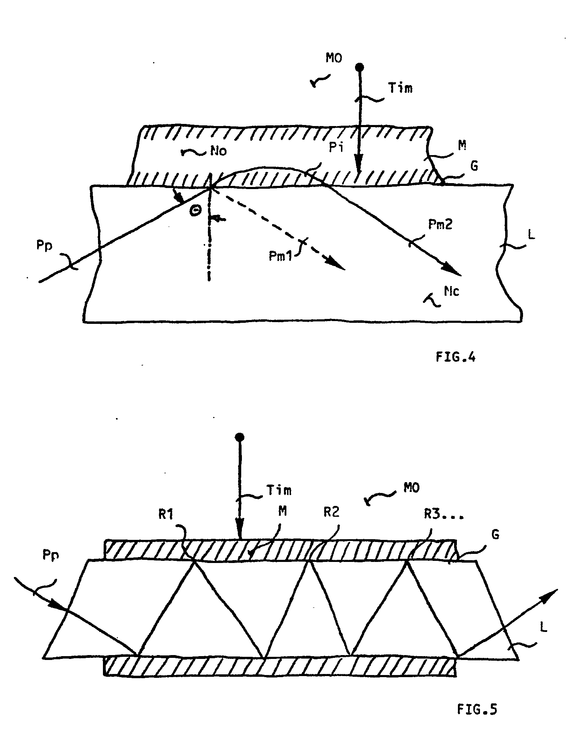

In Fig.4 erreicht ein Strahl Pp der Messtrahlung aus dem Lichtleiter L mit dem Brechungsindex Nc die Phasengrenze G zum Messraum M mit einem gegenüber Nc kleinerem Brechungsindex No. An der Phasengrenze G wird der Strahl Pp nicht in idealer Weise als Pm1 reflektiert, sondern dringt eine Strecke Pi in das Aussenmedium M ein. Dort nimmt die Amplitude nach der Beziehung

$$E = E_o e^{-z/dp}$$

mit

$$dp = \frac{\lambda}{N_o 2\pi (sin^2 \Theta - [N_o/N_c]^2)^{1/2}}$$

ab. Aus den Wellenfronten des Strahles Pp entsteht danach der Strahl Pm2.

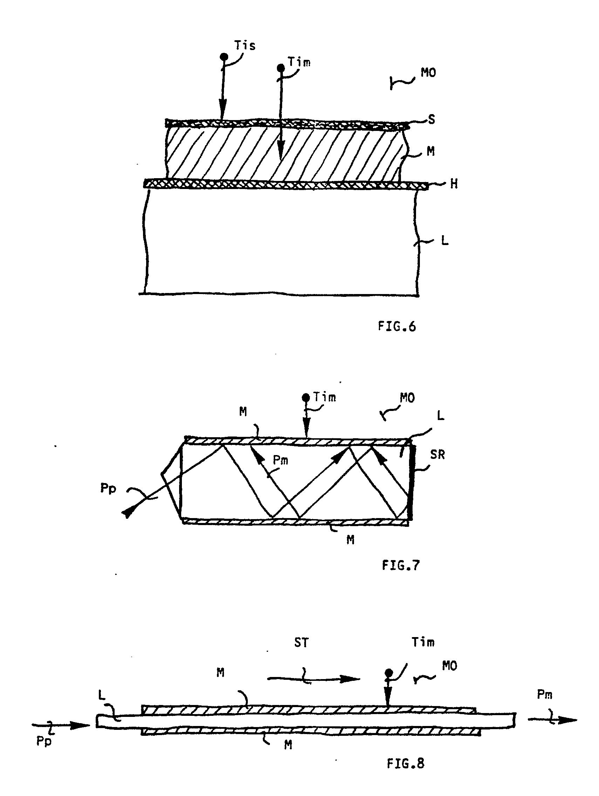

In einer Messanordnung gemäss Fig.5 verläuft die Messtrahlung Pp über eine längere Strecke im Lichtleiter L. Hier wiederholt sich bei jeder Reflexion R1,R2,... der Effekt der Grenzflächenabsorption im Messraum M mehrfach, so dass sich die durch die Konzentration bedingten Änderungen der Messtrahlung Pp verstärken.

Besteht der Messraum M aus Material mit Bindung oder mit hohem Löslichkeitskoeffizienten a für eine bestimmte Teilchenart T1m, dann steigt die Konzentration von T1m im Messraum M gegenüber dem Messobjekt MO.

Dadurch lässt sich die Konzentration im Messraum M um mehrere Grössenordnungen erhöhen.

Wird der Messraum M hydrophob ausgebildet, entsteht bei wässrigen Lösungen als Messobjekt eine Trennung des Aussenraumes in einen wasserfreien Messraum M und das Messobjekt MO. Andererseits diffundieren diffusionsfähige Stoffe solange in den Messraum M bis sie mit dem Messobjekt im Diffusionsgleichgewicht stehen. Der diffundierte und damit von Störsubstanzen abgetrennte Stoff kann nunmehr von innen, also vom Lichtleiter aus optisch analysiert werden.

Wegen der Rückdämmung des Wassers kann die Probe jetzt beispielsweise auch mit IR-Licht analysiert werden, weil die starke IR-Absorption durch die Wasserbanden entfällt.

Andererseits ist die Eindringtiefe wegen der grossen Wellenlängen hinreichend gross, sodass für viele Messprobleme ausreichende optische Weglängen im Messraum entstehen.

Das ist ein besonderer Vorteil, weil nunmehr die gesamte IR-Analysentechnik für den Messraum zur Anwendung kommen und auch ramanaktive Substanzen untersucht werden können.

Reste von Wasserdampf können aus dem Messraum ferngehalten werden, wenn dieser nach Fig.6 mit einer weiteren Schicht S, beispielsweise aus Tetrafluorethylen oder Mylar versiegelt ist. Solche Schichten sind nämlich für Wasserdampf (Tis) wenig durchlässig, für die zu messenden Teilchen Tim, etwa Sauerstoff, Narkosegas (Halothan) oder CO2 jedoch gut durchlässig, sodass sich diese Teilchenarten besonders gut in M konzentrieren und deshalb gut messen lassen. Die Schichten können mit einem Haftvermittler H mit dem Lichtleiter L verbunden sein.

Insbesondere bei Messung im IR-Bereich ist die Thermostatisierung der Messanordnung zweckmässig, weil dadurch die Eigenstrahlung der Anordnung konstant gehalten werden kann.

Der Lichtleiter kann gemäss Fig.7 als Tauchstab ausgebildet werden.

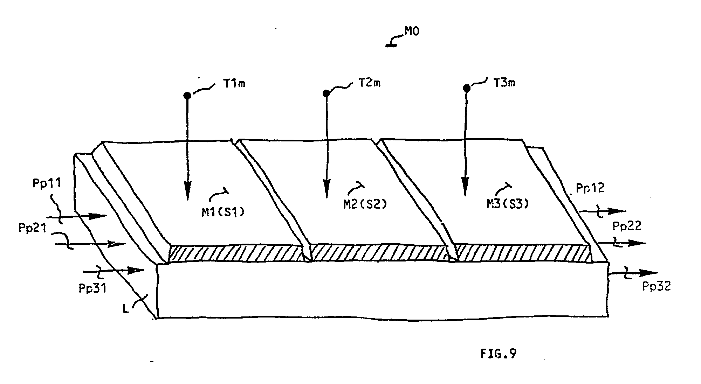

Zur Vermessung strömender Flüssigkeiten können nach Fig.8 die Totalreflexionen im Messraum M auf längeren Strecken des Lichtleiters L erfolgen, so dass ein langgestreckter, dünner Lichtleiter L verwendet werden kann, der zudem die Strömung ST wenig stört.

Sollen gleichzeitig mehrere Partikelarten T1m, T2m, T3m... durch Prüflichstrahlen Pp11, Pp21,... gemessen werden, können die Messräume M1(S1), M2(S2),.. gemäss Fig.9 gefeldert und aus verschieden teilchenselektiven Stoffen (S1,S2,..) aufgebaut oder mit diesen abgedeckt sein. Bei teilchenselektiven Stoffen kann nur eine bestimmte Teilchenart Tim durch Diffusion in den Messraum Mi(Si) eindringen. Sie wird durch Bestimmung der Änderung der zugeordneten Strahlen Pp11, Pp21.. nach Pp21, Pp22.. gemessen.

Ist einer der Prüflichtstrahlen P1i unabhängig vom Messvorgang, kann dieser als Referenzstrahl verwendet werden, indem die anderen gemessenen Intensitäten auf diese Strahlungsintensität bezogen wird. Dadurch können apparativ verursachte Intensitätsschwankungen rechernisch in bekannter Weise eliminiert werden.

Wenn bei Teilchenmessungen nur geringe Teilchenmengen zur Verfügung stehen, ist es zweckmässig, die Löslichkeit a des Messraumes klein zu halten und auf Empfindlichkeitssteigerung zu verzichten, um nicht eine das Messergebnis verfälschende Verarmung an zu messenden Teilchen zu provozieren.

**Patentansprüche**

1. Anordnung zur optischen Messung von Stoffkonzentrationen mit

   einer IR-Strahlungsquelle zur Erzeugung der Messstrahlung (Pp)

   einem Messraum (M) aus einem, fuer die zu messenden Teilchen (Tim) selektiv permeirbaren und fuer die Messstrahlung (Pp) transparenten Stoff, der so angeordnet ist, dass eine Diffusion der zu messenden Teilchen (Tim) in den Messraum erfolgen kann,

   einem Lichtleiter (L), der an seinen Seitenflaechen mit dem Messraum (M) umkleidet ist und der so angeordnet ist, dass die Messstrahlung (P) in den Lichtleiter (L) faellt und in ihm innerhalb des Grenzwinkels der Totalreflexion verlaeuft und

   einem Fotoempfanger (F) zur Untersuchung der aus dem Lichtleiter (L) wieder austretenden Messstrahlung (Pp).

2. Anordnung nach Anspruch 1, dadurch gekennzeichnet, dass das Material des Messraumes (M) fuer den zu messenden Stoff (T1m,T2m...) einen hohen Loeslichkeitskoeffizienten a aufweist.

3. Anordnung nach Anspruch 1, dadurch gekennzeichnet, dass das Material des Messraumes (M) fuer den zu messenden Stoff (T1m,T2m...) Bindungsstellen aufweist.

4. Anordnung nach Anspruch 2, dadurch gekennzeichnet, dass der Messraum aus organischen oder anorganischen Polymeren, beispielsweise aus Siliconen, aus PVC, aus Polystyrol oder Polypropylen besteht.

5. Anordnung nach einem der Ansprueche 1 bis 4, dadurch gekennzeichnet, dass eine Felderung des Messraumes (M1(s1) ,M2(S2) . ..) vorgesehen ist und dass die Messfelder (M1,M2..) selektiv fuer die zu messenden Teilchen (T1m,T2m..) ausgebildet sind.

6. Anordnung nach Anspruch 5, dadurch gekennzeichnet, dass die Messtrahlung verschiedene Wellenlaengen (P11, P12, . . . ) aufweist.

7. Anordnung nach Anspruch 6, dadurch gekennzeichnet, dass eine der Wellenlaengen (P1i) eine Referenzwellenlaenge ist .

8. Anordnung nach einem der Ansprueche 1 bis 7, dadurch gekennzeichnet, dass der Messraum (M) hydrophob ausgebildet ist.

9. Anordnung nach einem der Ansprueche 1 bis 8, dadurch gekennzeichnet, dass der Messraum (M) mit mindestens einer weiteren (S) Schicht zur Ausschaltung von Rueckwirkungen aus dem Messgut (MO) versehen ist.

10. Anordnung nach Anspruch 9, dadurch gekennzeichnet, dass die Schicht (S) aus Tetrafluorethylen oder Mylar besteht.

11. Anordnung nach Anspruch 9, dadurch gekennzeichnet, dass die Schicht (S) aus wasserunloeslichen Loesungsmitteln, wie beispielsweise Dioctylphtalat, besteht.

12. Anordnung nach Anspruch 1, dadurch gekennzeichnet, dass der Lichtleiter (L) als ein mit einem Spiegel (SR) abgeschlossener Tauchstab ausgebildet ist.

13. Anordnung nach Anspruch 5 dadurch gekennzeichnet, dass die Messraeume (M1(S1) ,M2(S2) . .) mit teilchenselektiven Stoffen abgedeckt sind.

14. Anordnung nach Anspruch 9, dadurch gekennzeichnet, dass die Schichten (S,M) durch Haftvermittler mit (H) dem Lichtleiter (L) verbunden sind.

15. Anordnung nach einem der Ansprueche 1 bis 14, dadurch gekennzeichnet, dass das Material des Messraumes (M) die Wellenlange der Absorption der zu messenden Teilchen (Tim) verschiebt.

16. Anordnung nach einem oder mehreren der Ansprueche 1 bis 15 dadurch gekennzeichnet, dass der, Messraum thermostatisiert ist.

17. Anordnung nach Anspruch 1, dadurch gekennzeichent, dass der den Messraum (M) bildende Stoff in einem ein poroeses Netzwerk bildenden Stoff angeordnet ist.

18. Anordnung nach Anspruch 1, dadurch gekennzeichnet, dass der Messraum (M) fuer die zu messenden Teilchen selektive Carrier aufweist.

19. Anordnung nach Anspruch 1, dadurch gekennzeichnet, dass der Lichtleiter (L,Lp,Lm) aus Thalliumbromid-jodid oder Calciumfluorid besteht.

## Claims

1. A device for the optical measurement of material concentration incorporating
an IR light source for production of the measuring beam (Pp)
a measuring chamber (M) made of a material which is selectively permeable to the particles to be measured (Tim) and which is transparent to the measuring beam (Pp), which is so arranged that diffusion of the particles to be measured (Tim) can occur in the measuring chamber,
an optical conductor (L) which is sheathed on its side surfaces by the measuring chamber (M) and which is so arranged that the measuring beam (P) is incident on the optical conductor (L) and passes along it within the critical angle of total reflection, and
a photoreceiver (F) for the detection of the measuring beam (Pp) emanating from the optical conductor (L).

2. A device according to claim 1, characterised in that the material of the measuring chamber (M) has a high absorption coefficient a for the material to be measured (T1m, T2m...).

3. A device according to claim 1, characterised in that the material of the measuring chamber (M) has attachment locations for the material to be measured (T1m, T2m...).

4. A device according to claim 2, characterised in that the measuring chamber consists of organic or inorganic polymers, for example silicon, PVC, polystyrene or polypropylene.

5. A device according to any one of claims 1 to 4, characterised in that sectioning of the measuring chamber (M1(s1), M2(S2) ...) is provided and that measuring fields (M1, M2 ..) are formed selectively for the particles to be measured (T1m, T2m...).

6. A device according to claim 5, characterised in that the measuring beam has different wavelengths (P11, P12, ...).

7. A device according to claim 6, characterised in that one of the wavelengths (P1i) is a reference wavelength.

8. A device according to any one of claims 1 to 7, characterised in that the measuring chamber (M) is hydrophobic.

9. A device according to any one of claims 1 to 8, characterised in that the measuring chamber (M) is provided with at least one further layer (S) for the exclusion of reactions from the object to be measured (MO).

10. A device according to claim 9, characterised in that the layer (S) consists of tetrafluoroethylene or mylar.

11. A device according to claim 9, characterised in that the layer (S) consists of water-insoluble solvents, such as for example dioctylphthalate.

**12.** A device according to claim 1, characterised in that the optical conductor (L) is constructed as an immersion rod shut off by a mirror (SR).

**13.** A device according to claim 5, characterised in that the measuring chambers (M1(S1), M2(S2)..) are covered with particle-selective materials.

**14.** A device according to claim 9, characterised in that the layers (S, M) are connected to the optical conductor (L) by binding means (H).

**15.** A device according to one of claims 1 to 14, characterised in that the material of the measuring chamber (M) shifts the absorption wavelength of the particles to be measured (Tim).

**16.** A device according to one or more of claims 1 to 15, characterised in that the measuring chamber has a thermostat.

**17.** A device according to claim 1, characterised in that the material forming the measuring chamber (M) is arranged in a material forming a porous network.

**18.** A device according to claim 1, characterised in that the measuring chamber (M) has a selective carrier for the particles to be measured.

**19.** A device according to claim 1, characterised in that the optical conductor (L, Lp, Lm) consists of thallium bromide iodide or calcium fluoride.

**Revendications**

**1.** Installation pour la mesure optique de la concentration de substances comprenant:
une source de rayonnement à rayons infrarouges pour la production du rayonnement de mesure (P$_p$),
une chambre de mesure (M) en matériau, sélectivement perméable aux particules à mesurer (Tim) et transparent aux radiations de mesure (P$_p$), agencée de telle sorte qu'une diffusion des particules à mesurer (Tim) puisse se faire dans la chambre de mesure,
un câble optique (L) entouré sur ses faces latérales par la chambre de mesure (M) et agencé de telle sorte que le rayonnement de mesure (P) parvienne au câble optique (L) et circule dans celui-ci à l'intérieur de l'angle limite de la réflexion totale, et
un photorécepteur (F) pour l'analyse du rayonnement de mesure (Pp) qui ressort du câble optique (L).

**2.** Installation selon la revendication 1, caractérisée en ce que le matériau de la chambre de mesure (M) des substances à mesurer (T1m, T2m...) possède un coefficient de solubilité élevé.

**3.** Installation selon la revendication 1, caractérisée en ce que le matériau de la chambre de mesure (M) des substances à mesurer (T1m, T2m...) comprend des points de liaison.

**4.** Installation selon la revendication 2, caractérisée en ce que la chambre de mesure est composée de polymères organiques ou inorganiques, tels que des silicones, du polychlorure de vinyle, du polystyrène ou du polypropylène par exemple.

**5.** Installation selon l'une quelconque des revendications 1 à 4, caractérisée en ce qu'il est prévu une subdivision par champs de la chambre de mesure ( M1(s1), M2(s2)...) et que les champs de mesure (M1, M2...) sont agencés sélectivement selon les particules à mesurer (T1m, T2m...).

**6.** Installation selon la revendication 5, caractérisée en ce que le rayonnement de mesure comprend différentes longueurs d'onde (P11, P12...).

**7.** Installation selon la revendication 6, caractérisée en ce que l'une des longueurs d'onde (P1i) est une longueur d'onde de référence.

8. Installation selon l'une quelconque des revendications 1 à 7, caractérisée en ce que la chambre de mesure (M) est hydrophobe.

9. Installation selon l'une quelconque des revendications 1 à 8, caractérisée en ce que la chambre de mesure (M) est pourvue d'une couche supplémentaire (S) au moins afin d'exclure les réactions provenant du matériau mesuré (MO).

10. Installation selon la revendication 9, caractérisée en ce que la couche (S) est composée de tétrafluoréthylène ou de Mylar.

11. Installation selon la revendication 9, caractérisée en ce que la couche (S) est composée d'un solvant non soluble dans l'eau tel que du dioctylphtalate.

12. Installation selon la revendication 1, caractérisée en ce que le câble optique (L) est agencé en forme de tige immergée terminée par un miroir (SR).

13. Installation selon la revendication 5, caractérisée en ce que les chambres de mesure ( M1(S1), M2(S2)-...) sont revêtues de matériaux sélectifs de particules.

14. Installation selon la revendication 9, caractérisée en ce que les couches (S, M) sont reliées au câble optique (L) par des agents de pontage (H).

15. Installation selon l'une quelconque des revendications 1 à 14, caractérisée en ce que le matériau de la chambre de mesure (M) décale la longueur d'onde d'absorption des particules à mesurer (Tim).

16. Installation selon l'une quelconque des revendications 1 à 15, caractérisée en ce que la chambre de mesure est commandée par thermostat.

17. Installation selon la revendication 1, caractérisée en ce que le matériau constituant la chambre de mesure (M) est agencé dans un matériau constituant un réseau poreux.

18. Installation selon la revendication 1, caractérisée en ce que la chambre de mesure (M) comporte des porteurs sélectifs pour les particules à mesurer.

19. Installation selon la revendication 1, caractérisée en ce que le câble optique (L, Lp, Lm) est composé de iodurebromure de thallium ou de fluorure de calcium.

FIG.1

FIG.1a

FIG.2

FIG.3

FIG.4

FIG.5

FIG.6

FIG.7

FIG.8

FIG.9